Europäisches Patentamt

European Patent Office

Office européen des brevets

(11) Numéro de publication: **0 245 132**
**A1**

# DEMANDE DE BREVET EUROPEEN

(21) Numéro de dépôt: 87400759.4

(22) Date de dépôt: 06.04.87

(51) Int. Cl.⁴: **A 61 M 5/00**
A 61 B 5/04, A 61 B 5/06

(30) Priorité: 16.04.86 FR 8605462

(43) Date de publication de la demande:
11.11.87 Bulletin 87/46

(84) Etats contractants désignés:
AT BE CH DE ES FR GB GR IT LI LU NL SE

(71) Demandeur: CELSA COMPOSANTS ELECTRIQUES SA
Usine du Château d'Eau
F-86420 Monts Sur Guesnes (FR)

RDM MEDICAL, Société dite (société à responsabilité
limitée)
"Les Saules" - RN 19
F-77170 Brie Comte Robert (FR)

(72) Inventeur: Lehmann, Gérard
6, rue de Poitiers
Avanton F-86170 Neuville de Poitou (FR)

Metais, Joel
La Cottencière Berthegon
F-86420 Monts sur Guesnes (FR)

Meunier, Jean- François
8, rue Bernard Naudin
F-93160 Noisy le Grand (FR)

Gautier, Jean-Philippe
91 bis, avenue du Général Leclerc
F-77330 Ozoir La Ferrière (FR)

(74) Mandataire: Lerner, François
5, rue Jules Lefèbvre
F-75009 Paris (FR)

(54) Capsule distributrice de drogue implantable et dispositif facilitant son utilisation.

(57) L'invention concerne une capsule distributrice de drogue implantable ainsi qu'un procédé et un dispositif facilitant seon utilisation.

Selon l'invention, on prévoit à l'intérieur du volume (5) de la capsule (3) un organe de détection de proximité (7) de l'extrémité de l'aiguille (1) d'injection, lequel organe de détection est relié à un dispositif (32) de signalisation externe qui signale le moment où cette proximité est réalisée.

L'invention s'applique, en particulier, à l'injection, dans des endroits localisés, de drogues diverses.

FIG.1

**Description**

## CAPSULE DISTRIBUTRICE DE DROGUE IMPLANTABLE ET DISPOSITIF FACILITANT SON UTILISATION

L'invention a pour objet un dispositif facilitant l'utilisation d'une capsule distributrice de drogue implantable.

Pour le traitement de certaines affections graves, on est amené à effectuer de fréquentes injections de drogues diverses. Fréquemment, ces injections doivent se faire par voie veineuse ou artérielle, ou encore par d'autres voies, de façon à assurer la distribution locale et précise des drogues traitantes. Pour éviter la mise en place et le retrait, à chaque opération d'injection, d'un cathéter, on a eu recours depuis déjà de nombreuses années à des capsules distributrices de drogues implantables, mises en place sous la peau du patient en un endroit adapté et dont le volume interne est en communication avec un conduit laissé en place dans le corps et débouchant à l'endroit où les injections doivent être effectuées. La capsule présente une paroi perforable à travers laquelle il est possible d'injecter au moyen d'une aiguille la drogue à distribuer, cette paroi se refermant automatiquement au retrait de l'aiguille.

D'une façon générale, et comme on le comprend, dans ce domaine d'application, un grave problème de fond se présente concernant l'assurance qui doit être donnée au praticien de l'introduction correcte de l'aiguille dans le volume intérieur de la capsule distributrice. En effet, certaines des drogues utilisées peuvent être d'une efficacité redoutable et entraîner la mort du patient ou des accidents graves si l'injection de drogue, par erreur, ne s'effectue pas à l'intérieur du volume de la capsule, et se répand, par exemple, entre cette capsule et la peau qui la recouvre.

Dans un certain nombre de dispositifs de repérages d'une bonne introduction de l'aiguille de la seringue d'injection dans le volume de la capsule distributrice de drogues, cette même capsule est mise en contact par sa paroi extérieure avec le corps du patient et comprend une paroi interne conductrice d'électricité formant organe de détection de proximité de l'extrémité de cette aiguille. En outre, il est connu de munir le dispositif de repérage, d'un appareil de signalisation externe relié à l'organe de détection précité et qui répond à l'état de fermeture à travers le corps du patient, d'un circuit électrique qui comprend la paroi interne conductrice de la capsule précitée ainsi que l'aiguille.

De tels dispositifs présentent néanmoins un certain nombre d'inconvénients.

En effet, on s'est aperçu, que, en corrélation avec le problème de fond concernant l'assurance d'une bonne introduction de l'aiguille, se posaient des difficultés pratiques d'application et d'utilisation des dispositifs connus. Plus précisément, il a été remarqué que des déclenchements intempestifs de l'appareil de signalisation qui avertit le praticien de l'introduction correcte de l'aiguille survenaient parfois alors que cette aiguille n'était pas encore en contact avec le fond de la capsule. Une faible variation d'impédance, trop rapide, pouvait déclencher l'appareil, entraînant, comme on le comprend, un danger réel pour le malade.

L'invention a notamment pour objet de résoudre cette difficulté, en liaison avec le problème de fond soulevé précédemment, en permettant au praticien de déterminer à coup sûr le moment où l'aiguille d'injection est effectivement et correctement introduite, jusqu'à la paroi interne conductrice, à l'intérieur du volume de la capsule; et ce sans risque d'un déclenchement intempestif trop rapide de l'appareil de signalisation qui informe le praticien qu'il peut injecter la drogue.

Cet objet est atteint selon l'invention en prévoyant que le circuit électrique du dispositif de repérage précité comprend, outre la paroi interne conductrice de la capsule et l'aiguille d'injection,

- un générateur de courant alternatif dont l'entrée est reliée au pôle positif d'une source de tension continue,

- un montage du type redresseur/filtre, ledit montage étant relié :

. par une première borne d'entrée, à la sortie dudit générateur de courant alternatif,

. par une seconde borne, au pôle négatif de ladite source de courant continu,

- au moins un comparateur relié

. en sortie, audit appareil de signalisation,

. en entrée, à une troisième borne de sortie du montage redresseur/filtre et à une unité de comparaison destinée à fournir une tension de référence,

ledit comparateur étant soumis à la différence de tension entre celle délivrée par ledit montage redresseur/filtre et celle délivrée par la dite unité de comparaison, et étant alimenté, avec ledit appareil de signalisation, par ladite source de courant continu,

- une électrode de surface disposée sur la peau du patient, ladite électrode étant reliée, dans l'état fermé du circuit, en série avec ladite aiguille, le corps du patient et/ou ladite paroi interne de la capsule vers la borne d'entrée dudit montage redresseur/filtre.

Selon une caractéristique préférée, l'unité de comparaison précitée comprend :

- une horloge de séquencement dont une borne d'entrée est reliée à la borne de sortie du montage redresseur/filtre,

- deux commutateurs placés sur la commande de l'horloge,

- un condensateur ou analogue qui se décharge vers une entrée du comparateur dans une position fermée de l'un des commutateurs, et

- un pont diviseur de tension qui assure la charge du condensateur dans la position fermée de l'autre commutateur, les deux commutateurs travaillant en opposition de phase.

De cette façon, on évite que le dispositif prenne en compte les petites variations d'impédance dont l'ampleur est fonction essentiellement de la vitesse d'introduction de l'aiguille d'injection.

En d'autres termes, on assure, grâce à l'invention, le déclenchement de l'appareil de signalisation uniquement lorsque l'impédance chute brusquement et de façon relativement importante, c'est-à-dire essentiellement uniquement lors du contact entre l'extrémité de l'aiguille d'injection et la paroi interne conductrice de la capsule.

On notera également que la prévision, dans l'invention, d'un générateur de courant alternatif permet de résoudre un problème délicat concernant l'efficacité du dispositif dans son ensemble. En effet, du fait de la mise en place de la capsule sous la peau et de son utilisation, il se crée autour de la capsule des tissus fibreux qui perturbent le passage de l'énergie électrique, et en particulier celle qui serait délivrée par un générateur ou une source de courant continu. Un fonctionnement en courant alternatif résoud cette difficulté supplémentaire.

En outre, un tel générateur de courant est tout à fait adapté en ce qu'il permet de contrôler l'énergie électrique qui passe dans le corps du patient en ne vérifiant qu'une seule variable ; le courant.

L'invention et sa mise en oeuvre apparaîtront plus clairement à l'aide de la description qui va suivre faite en référence aux dessins annexés illustrant des modes de mise en oeuvre. Dans ces dessins :

    - la figure 1 est un schéma synoptique d'un montage pouvant être utilisé conformément à l'invention,

    - la figure 2 illustre un montage quelque peu complété par rapport à celui de la figure 1, et

    - la figure 3 montre, en coupe transversale, une capsule distributrice utilisable conformément à l'invention.

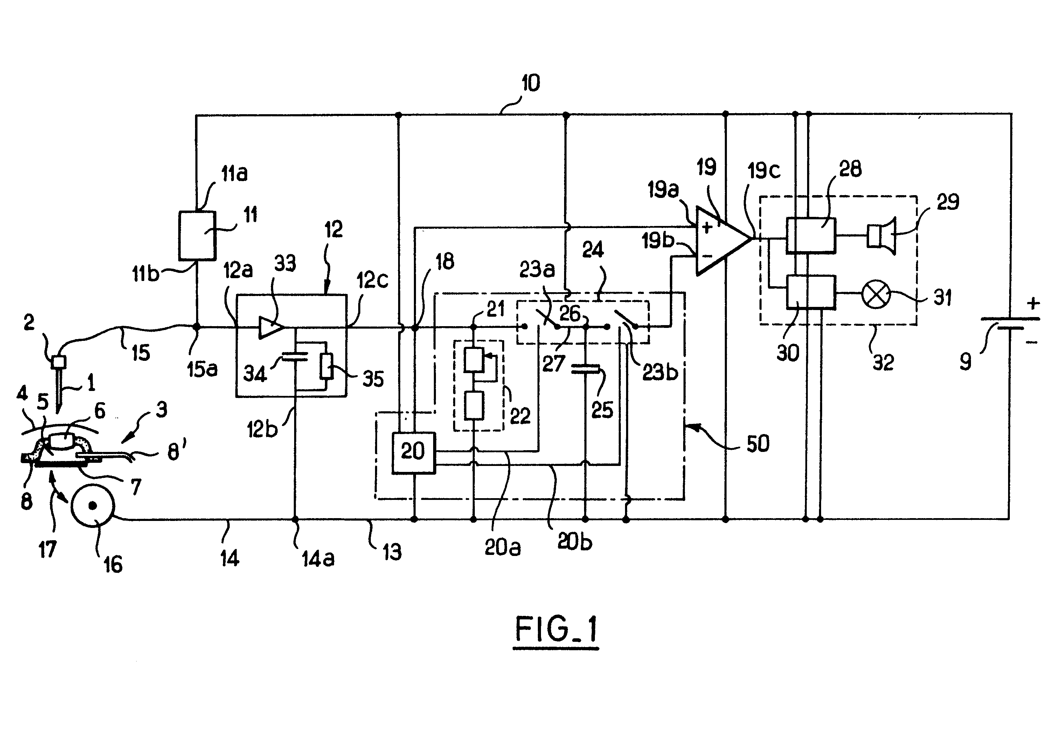

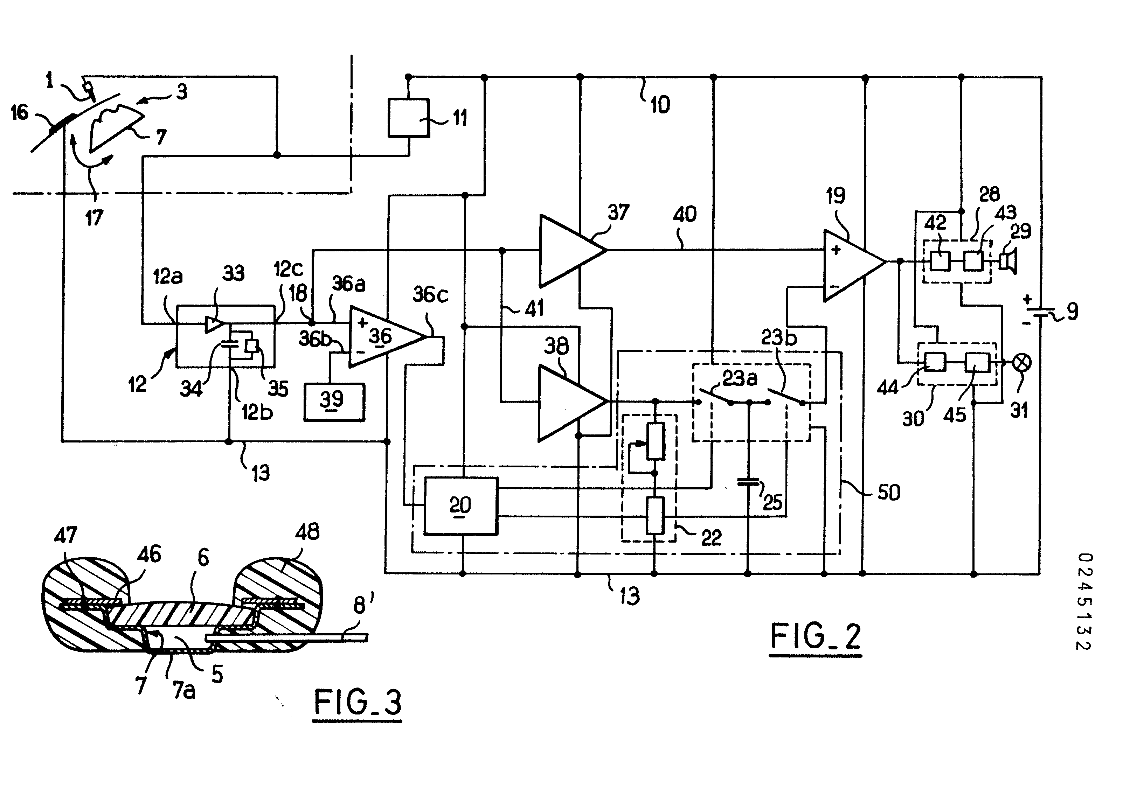

On notera dès à présent que sur les figures 1 et 2, pour faciliter la lecture des dessins, les points de liaison ou noeuds du circuit ont été repérés par un rond plus marqué.

En se reportant tout d'abord à la figure 1, on a indiqué en 1 l'aiguille avec sa tête 2 d'adaptation sur tout système approprié (non représenté) d'injection et/ou de ponction. En face, on a repéré en 3 une capsule implantable sous la peau 4 d'un patient, cette capsule comprenant, fermant son volume interne 5, une paroi 6 auto-obturante d'un type en soi connu, notamment en matériau plastique siliconé. La paroi interne de fond 7 de la capsule est métallique, tandis que le reste de la capsule, et notamment la paroi latérale 8, est en matériau isolant électrique, tel qu'un plastique de qualité appropriée. Le volume 5 de la capsule communique avec un conduit 8' par lequel sont injectées les drogues à introduire, et par lequel peuvent également être effectués des soutirages pour analyses ou ponctions. Le dispositif de l'invention, comprend, en outre, une source 9 de courant électrique continu de qualité convenable (tension/intensité). Conformément à l'invention, l'entrée 11a d'un générateur de courant alternatif 11 est relié au pôle positif de la source 9 de courant continu par l'intermédiaire d'une ligne d'alimentation 10. La sortie 11b de ce même générateur 11 est reliée à une borne d'entrée 12a d'un montage 12 formant redresseur/filtre.

Le montage 12 comprend, tel qu'illustré, une diode 33, ou analogue, formant redresseur branchée entre l'entrée 12a et la sortie 12c, un condensateur 34 relié d'un côté à la ligne d'alimentation de l'unité, vers la cathode de la diode, et de l'autre, en sortant par la borne 12b, au pôle négatif de la source 9, par l'intermé diaire de la ligne de masse 13 du circuit. En parallèle aux bornes du condensateur, est montée une résistance 35 relativement forte.

La diode est montée passante vers la sortie 12c du montage.

En 17, la double flèche indique le trajet électrique de résistance réduite existant à travers le corps du patient entre une électrode de surface 16 reliée en 14a, par un conducteur 14, à la ligne de masse 13, et la paroi interne de fond métallique 7 de surface relativement importante de la capsule 3. Un conducteur 15 permet par ailleurs de relier, en 15a, la tête 2 de l'aiguille 1 entre la sortie 11b du générateur 11 de courant alternatif et la borne d'entrée 12a du montage 12. Ainsi, lorsque l'aiguille 1 vient en contact au moins avec la peau 4 du patient, le circuit se trouve fermé en série sur l'aiguille, l'électrode de surface, et le corps du patient.

A la borne 12c de sortie de l'unité 12 redresseur/filtre précitée, sont reliés, au niveau d'un noeud 18, d'une part l'entrée positive 19a d'un comparateur 19 lui-même relié à l'appareil de signalisation 32 et, d'autre part, une unité de comparaison 50 destinée à fournir à l'entrée négative 19b de ce même comparateur, une tension variable comme on le verra ultérieurement. L'unité 50 comprend une horloge 20, un diviseur de tension 22, un condensateur 25 et deux commutateurs 23a, 23b. La borne d'entrée de l'horloge 20 de séquencement est reliée au noeud 18, tandis qu'en 21, est reliée, toujours à la borne 12c de l'unité 12, l'un des pôles du diviseur de tension 22. L'autre pôle de ce même diviseur est en liaison avec la ligne 13 de masse.

Les deux commutateurs 23a, 23b sont placés sous la dépendance de l'horloge 20. Ils sont reliés d'une part l'un à l'autre au moyen d'un conducteur 27 et, d'autre part, au niveau de leur borne restante respective, pour le commutateur 23a, à la ligne de sortie du commutateur 12, en 21, et, pour le commutateur 23b, à l'entrée négative 19b du comparateur 19.

Sur la ligne de liaison 27 d'une borne de chacun des deux commutateurs est branché, en 26, un pôle du condensateur 25 dont l'autre pôle est relié à la ligne de masse 13.

Dans le cas envisagé, les commutateurs sont du type "tout ou rien", ouvert ou fermé. Sur les figures, ils ont été représentés tous les deux en position ouverte, bien qu'ils aient été prévus pour fonctionner en opposition de phase.

L'action de commande ou de contrôle de l'horloge 20 sur les deux commutateurs 23a, 23b, s'effectue par l'intermédiaire de deux conducteurs respectivement 20a, 20b reliés à deux bornes de sortie de l'horloge 20.

En ce qui concerne l'appareil de signalisation 32, on remarquera, tel qu'illustré, que la sortie 19c du comparateur 19 est reliée en parallèle, d'une part à un appareil 28 de mise en forme d'un signal sonore qui pourra activer un dispositif sonore 29, et, d'autre part, à un appareil de mise en forme 30 du signal qui pourra activer un voyant limineux 31.

Afin d'assurer le fonctionnement du circuit qui vient d'être décrit, la source 9 de courant continu, qui peut être notamment une simple pile galvanique, alimente en parallèle, par l'intermédiaire de la ligne d'alimentation 10 et de la ligne de masse 13, les deux appareils 28, 30 de mise en forme du signal, le comparateur 19, l'unité 24 comprenant les deux commutateurs 23a, 23b et l'horloge de séquencement 20.

Le fonctionnement du dispositif de l'invention dont le circuit de montage vient d'être décrit se déduit clairement de ce qui précède.

Dans l'état illustré à la figure où l'aiguille 1 est éloignée de la paroi 7 métallique conductrice de la capsule 3, le circuit électrique, à travers l'aiguille et la capsule est ouvert, une résistance quasiment infinie étant présente entre ses bornes.

Lorsque l'aiguille 1 est enfoncée à travers la peau 4 du patient, la résistance diminue, le circuit électrique étant fermé à travers le corps et la peau du patient. Cependant, cette résistance est encore très forte du fait du contact ponctuel limité entre l'aiguille 1 et la peau 4 à l'endroit où cette même aiguille traverse la peau. A titre d'exemple, on notera que cette résistance peut être de l'ordre de 30 000 Ohms.

Enfin, lorsque l'aiguille a perforé la paroi 6 et vient en contact avec le fond métallique 7 de la capsule, la résistance diminue considérablement, ne dépassant pas par exemple 1 000 Ohms. Au niveau du comparateur 19, c'est cette variation d'impédance, qui est mise à profit par le comparateur pour déclencher les signaux.

Bien entendu, le dispositif est temporisé de façon que le signal sonore et le signal limineux ait une durée de temps suffisante pour qu'il n'y ait au niveau de l'utilisateur aucun doute sur la présence du signal.

En fait, le comparateur ne déclenchera les signaux que si la tension qui arrive à sa borne positive 19a est inférieure à une tension mémorisée précédente arrivant à sa borne négative 19b.

En effet, dès la fermeture du circuit et dès la mise sous tension de la source 9, l'horloge 20 est déclenchée et commande alternativement à chaque battement le commutateur 23a ou le commutateur 23b. Parallèlement, le générateur 11 de courant alternatif alimente le montage 12 (redresseur 33/Filtre 34,35) qui reçoit en 12a un courant alternatif, le redresse, le filtre, ou l'intègre, et délivre en sortie entre ses bornes 12c, 12b une tension continue U fonction du courant qui traverse l'impédance (ou résistance) au niveau du corps du patient et/ou de la capsule. La tension U se retrouve aux bornes du pont diviseur 22 et à l'entrée positive 19a du comparateur 19.

Soit φ la phase de battement de l'horloge qui commande, à la fermeture, le commutateur 23a et $\overline{\varphi}$ la phase qui commande, également à la fermeture, le commutateur 23b. On rapelle que de préférence, les deux commutateurs travaillent en opposition de phase.

En phase φ, le commutateur 23a est fermé, tandis que 23b est ouvert. Le condensateur 25 se charge de la tension délivrée par le pont diviseur 22, c'est-à-dire d'une fraction de la tension U, soit $\frac{U}{X}$ avec X: rapport du pont diviseur.

En phase $\overline{\varphi}$ le commutateur 23b est fermé, tandis que 23a est ouvert. Le condensateur 25 se décharge alors vers l'entrée 19b négative du comparateur 19, lequel compare alors la valeur d'une tension U' qui vient d'être recueillie au niveau de sa borne positive 19a à la valeur de tension mémorisée $\frac{U}{X}$ reçue à sa borne négative 19b. En d'autre terme, le comparateur 19 effectue une comparaison entre une tension proportionnelle au courant qui vient de traverser le corps du patient et une valeur fractionnaire précédente de cette tention qui a été stockée dans le condensateur.

On peut notamment prévoir de régler le pont diviseur 22 avec X = 4. La tension qu'il délivrera sera donc $\frac{U}{4}$, avec une tension U à l'entrée.

Le comparateur 19 déclenchera donc les signaux s'il reçoit une tension U' inférieure à $\frac{U}{4}$, c'est-à-dire si un éffondrement important de l'impédance survient, indiquant le contact entre l'aiguille 1 et fond 7 de la capsule et donc une introduction correcte de l'aiguille.

L'horloge 20 pourra être réglée à environ cent fois la vitesse v de pénétration de l'aiguille dans le site. On peut considérer v de l'ordre de 100 ms et donc φ (ou $\overline{\varphi}$) égale à environ 1 ms. Ainsi, on évite que la vitesse d'introduction de l'aiguille puisse venir perturber le fonctionnement du dispositif et entraîner un déclenchement intempestif des signaux.

On se reportera maintenant à la figure 2 pour voir illustré un mode de réalisation préféré et quelque peu plus complet du montage électrique représenté à la figure 1.

Le circuit de la figure 2 comprend tous les composants qui ont été présentés en référence à la figure 1 et qui, en conséquence, ont reçu la même numérotation et ne seront pas à nouveau décrits.

En particulier, on retrouve, outre l'aiguille 1, la capsule 4 et l'électrode de surface 16, le générateur 11 de courant alternatif, le montage 12 redresseur/filtre, l'horloge 20, le pont diviseur 22 de tension, les commutateurs 23a et 23b, le comparateur 19 et l'appareil 32 de signalisation, ainsi que la source 9 de courant continu.

Au montage, on a ajouté un second comparateur 36 et deux suiveurs de tension respectivement 37 et 38.

En outre, on a illustré plus en détail, les appareils 28, 30, de mise en forme du signal.

De façon plus spécifique, le second comparateur 36 a été disposé entre le noeud 18 et la borne d'entrée de l'horloge 20. Plus précisément, son pôle d'entrée positif 36a est en liaison avec la borne 12c de sortie du montage 12 redresseur/filtre, tandis que son pôle d'entrée négatif 36b est relié à une unité 39 de comparaison ou unité de référence, en soi connue, délivrant une tension fixe.

Le comparateur 36 est réglé de façon qu'il commande le déclenchement de l'horloge 20 lorsque la tension appliquée à sa borne 36a tombe en dessous d'un seuil déterminé pris par rapport à la tension régnant à sa borne 36b qui reçoit la tension de référence. En pratique la comparateur 36 sera de préférence réglé pour se déclencher dès le contact de l'aiguille 1 avec la peau 4 du patient. Il améliore la fiabilité du dispositif.

Quant aux suiveurs de tension 37 et 38, ils sont respectivement montés sur la ligne d'alimentation 40 de la borne positive du comparateur 19 et sur celle 41 du diviseur de tension 22, de telle sorte que leur entrée respective soit soumise à la tension U (ou U') délivrée par le montage 12 redresseur/filtre.

En ce qui concerne les appareils 28, 30 de mise en forme, ils comprennent chacun un circuit monostable 42, 44 commandé par le comparateur 19 et, en série, un circuit d'adaptation 43, 45.

Bien entendu, le comparateur 36, les deux suiveurs 37, 38 et les composants des appareils 28, 30, sont alimentés en parallèle par la source de courant continu 9.

A titre d'utilisation pratique, on notera que l'on peut prévoir d'utiliser comme comparateur 36, le modèle LD 161 fabriqué par la Société INTERSIL. Ce même modèle pourrait également convenir pour le comparateur 19.

Les deux suiveurs de tension 37, 38 pourraient être du type TLO81 fabriqué par la SOciété TEXAS INSTRUMENTS.

Au niveau du choix des composants, on peut retenir un condensateur 25 d'environ 100 nF, un condensateur 34 d'environ 10 nF, une résistance 35 sensiblement égale à 2 M $\Omega$ un générateur 11 de courant alternatif délivrant environ 100 µA avec une source de courant 9 d'environ $\pm$ 9 V.

On va maintenant commenter la figure 3 sur laquelle on a illustré la réalisation d'une capsule particulièrement adaptée à l'usage précédemment décrit.

La capsule comprend essentiellement un fond 7 en métal embouti, par exemple un acier inox ou du titane. Le fond métallique a la forme représentée à la figure 3 d'une cuvette, le volume interne 5 de la capsule étant refermé par la paroi perforable 6 auto-obturante placée sur la cuvette. Le disque 6 peut être maintenu en place au moyen d'une rondelle métallique 46 soudée comme indiqué en 47 vers le bord externe de la paroi 7 formé en cuvette. L'ensemble est alors enrobé d'un matériau plastique surmoulé 48 isolant électriquement l'ensemble de la capsule du corps du patient, sauf la paroi centrale de fond 7a de la capsule qui viendra en contact avec le corps du patient, par exemple un os, sur lequel la capsule sera fixée. Enfin, le volume intérieur 5 de la cap sule communique par un tube métallique 8' convenablement soudé ou serti, traversant la paroi 7, le montage étant convenablement protégé par le plastique surmoulé 48. On note qu'avec une telle capsule, lorsqu'on vient perforer la paroi 6 au moyen de l'aiguille d'injection, il n'y a pas de risque d'obtenir un faux signal de contact si l'aiguille est mal dirigée et vient heurter la paroi isolante de l'enrobage 48 de la capsule.

Différentes variantes peuvent être apportées aux modes de réalisation décrits, tant dans la constitution de la capsule que dans la génération du signal indiquant l'introduction correcte et complète de l'aiguille dans le volume de la capsule jusqu'au fond métallique interne de celle-ci. Par exemple, on

pourrait envisager d'utiliser, dans le cas du montage illustré à la figure 1, au lieu du comparateur 19 et de l'unité de comparaison 50, le comparateur 36 précité avec son unité de comparaison 39.

Dans ce cas, l'appareil de signalisation 32 serait branché directement à la sortie du comparateur 36. Le reste du circuit serait identique à celui illustré.

Avec un tel montage, et comme on le comprend, la variation d'impédance liée à la pénétration de l'aiguille vers le fond conducteur de la capsule, serait mise à profit par le comparateur 36 pour déclencher, à un seuil déterminé, par exemple 2 000 Ohms, les signaux indicateurs de bonne introduction. Toutefois, dans ce cas, il n'y aurait, bien entendu, pas de comparaison entre une tension immédiate et une valeur précédente qui aurait été stockée pendant une courte période. En d'autres termes et en pratique, un tel dispositif, bien que tout à fait utilisable, ne permettrait pas de se dégager des incertitudes de déclenchement intempestif de l'appareil de signalisation liées à la vitesse de pénétration de l'aiguille.

## Revendications

1. - Dispositif de repérage de la bonne introduction de l'aiguille (1) d'une seringue d'injection dans le volume d'une capsule (3) distributrice de drogues implantable sous la peau (4) d'un patient, ladite capsule qui est mise en contact par sa paroi externe avec le corps du patient comprenant une paroi interne (7) conductrice d'électricité formant organe de détection de proximité de l'extrémité de ladite aiguille (1) et ledit dispositif étant muni d'un appareil (32) de signalisation externe, relié audit organe (7) de détection et répondant à l'état de fermeture à travers le corps du patient d'un circuit électrique comprenant ladite paroi interne (7) conductrice de la capsule et ladite aiguille (1), caractérisé en ce que ledit circuit électrique comprend, en outre,
- un générateur (11) de courant alternatif dont l'entrée (11a) est reliée au pôle plus (+) d'une source (9) de courant continu,
- un montage (12) du type redresseur (33)/filtre 34,35), ledit montage étant relié
. par une première borne (12a) d'entrée, à la sortie (11b) dudit générateur (11),
. par une seconde borne (12b) au pôle négatif (-) de ladite source (9),
- au moins un comparateur (19, 36) relié
. en sortie (19c, 36c) audit appareil de signalisation (32),
. en entrée (19a, 19b, 36a, 36b) à une troisième borne (12c) de sortie du montage (12) redresseur/filtre et à une unité (39, 50) de comparaison destinée à fournir une tension de référence,
ledit comparateur étant soumis à la différence de tension entre celle délivrée par ledit montage (12) redresseur/filtre et celle délivrée par ladite unité (39, 50) de comparaison, et étant alimenté, avec ledit appareil (32) de signalisation, par ladite source (9) de courant continu,

- une électrode (16) de surface disposée sur la peau (4) du patient, ladite électrode étant reliée, au pôle négatif de la source (9) et, dans l'état fermé du circuit, en série avec ladite aiguille (1), le corps du patient et/ou ladite paroi interne (7) de la capsule (3), vers la borne d'entrée (12a) dudit montage (12) redresseur/filtre.

2. - Dispositif selon la revendication 1, caractérisé en ce que le montage (12) redresseur/filtre comprend:

- un composant redresseur de courant tel qu'une diode (33) disposée sur la ligne d'alimentation joignant la borne (12a) d'entrée et la borne (12c) de sortie du montage,

- un condensateur (34) ou analogue relié d'une part à ladite ligne d'alimentation, au niveau de la cathode de ladite diode (33), et d'autre part, au pôle négatif de la source (9), et

- une résistance (35) montée en parallèle aux bornes dudit condensateur (34).

3. - Dispositif selon la revendication 1 ou la revendication 2, caractérisé en ce que ladite unité (50) de comparaison comprend :

- une horloge (20) de séquencement dont une borne d'entrée est reliée à la borne de sortie (12c) dudit montage (12) redresseur/filtre,

- deux commutateurs (23a, 23b) placés chacun sous la commande de ladite horloge (20) et reliés entre-eux (27) au niveau d'une de leurs bornes passantes,

. ledit premier commutateur (23a) étant, par son autre borne passante, relié à l'une des bornes d'un pont diviseur de tension (22), elle-même en liaison avec la borne de sortie (12c) dudit montage (12) redresseur/filtre, l'autre borne du pont diviseur étant reliée au pôle négatif de la source (9), et

. ledit second commutateur (23b) étant, quant à lui, également par son autre borne passante, relié à une entrée (19b) du comparateur (19),

l'horloge (20) et les deux commutateurs (23a, 23b), étant, en outre, alimentés en courant continu par ladite source (9),

- un condensateur (25) ou analogue, relié d'un côté au pôle négatif de ladite source (9) et, de l'autre, au niveau de la liaison (27) entre-eux des deux commutateurs (23a, 23b).

4. - Dispositif selon la revendication 3, caractérisé en ce qu'il comprend deux comparateurs (19, 36),

- le premier (19) étant relié respectivement par ses deux entrées (19a, 19b), à la borne de sortie (12c) dudit montage (12) redresseur/filtre, et à ladite autre borne passante du second commutateur (23b), la sortie (19c) de ce premier comparateur (19) étant directement en liaison avec ledit appareil de signalisation (32),

- le second comparateur (36) étant disposé entre la borne de sortie (12c) dudit montage (12) redresseur/filtre et la borne d'entrée de ladite horloge (20), l'unité de comparaison (39) à laquelle est reliée l'une (36b) des entrées de ce second comparateur (36) étant du type délivrant une tension fixe déterminée.

5. - Dispositif selon la revendication 3 ou la revendication 4, caractérisé en ce qu'il comprend deux suiveurs de tension (37, 38) disposés chacun entre la borne de sortie (12c) dudit montage (12) redresseur/filtre et l'entrée (19a, 19b) correspondante du comparateur (19) qui est soumis à la différence de tension entre celle délivrée par ledit montage (12) redresseur/filtre et celle délivrée par ladite unité (50) de comparaison, les deux suiveurs étant alimentés par ladite source (9) de courant continu.

6. - Dispositif selon l'une des revendications 1 à 5, caractérisé en ce que ladite capsule est formée par une paroi métallique conductrice (7) formant fond de la capsule sur laquelle est surmoulé le reste de la capsule en matériau plastique (48) peu conducteur comprenant en regard de la paroi de fond la cloison perforable étanche (6).

7. - Dispositif selon la revendication 6, caractérisé en ce que le fond métallique de la capsule est formé par une pièce (7) mise en forme par emboutissage ayant une forme de cuvette obturable à sa partie supérieure par la cloison perforable (6) formant couvercle, l'étanchéité et l'assemblage étant obtenus par surmoulage de l'ensemble par une résine plastique (48) de qualité appropriée.

8. - Dispositif selon la revendication 6 ou la revendication 7, caractérisé en ce qu'un embout métallique (8') est fixé audit fond métallique (7) reliant l'intérieur de la capsule au tube de distribution implanté.

**FIG.1**

0245132

FIG_2

FIG_3

0245132

Office européen
des brevets

**RAPPORT DE RECHERCHE EUROPEENNE**

Numéro de la demande

EP  87 40 0759

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 505 167  (BRESLER & McCORMICK)<br>* Pages 10-12; figures 1,4 * | 1 | A 61 M  39/02<br>A 61 B   5/04<br>A 61 B   5/06 |
| | --- | | |
| A | WO-A-8 302 063  (FISCHELL)<br>* Page 10, lignes 12-23;  figures 3,6 * | · 1 | |
| | --- | | |
| A | US-A-4 273 531  (HASEGAWA)<br>*  Colonne  1, ligne 63 - colonne 2, ligne 45; figures 1,2 * | 1 | |
| | --- | | |
| A | US-A-2 516 882  (L. KALOM)<br>* Colonne 2, lignes 3-8; figure 2 * | 1 | |
| | ----- | | |

DOMAINES TECHNIQUES
RECHERCHES (Int. Cl.4)

A 61 M
A 61 B

Le présent rapport de recherche a été établi pour toutes les revendications

| Lieu de la recherche | Date d'achèvement de la recherche | Examinateur |
|---|---|---|
| LA HAYE | 14-08-1987 | EHRSAM F.J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82